# EUROPEAN PATENT SPECIFICATION

(11) **EP 1 668 144 B1**
(45) Date of publication and mention of the grant of the patent: **17.08.2011**
(21) Application number: 04765086.6
(22) Date of filing: 10.09.2004
(51) Int. Cl.: C12N 15/87, C12N 15/11, A61K 31/713

(54) **NUCLEOTIDE SEQUENCES PROMOTING THE TRANS-MEMBRANE TRANSPORT OF NUCLEIC ACIDS**
DEN TRANSMEMBRANTRANSPORT VON NUKLEINSÄUREN FÖRDERNDE NUKLEOTIDSEQUENZEN
SEQUENCES NUCLEOTIDIQUES PROMOUVANT LE TRANSPORT TRANSMEMBRANAIRE D'ACIDES NUCLEIQUES

(30) Priority: 11.09.2003 EP 03020437
(43) Date of publication of application: 14.06.2006
(73) Proprietor: Universitätsklinikum Schleswig-Holstein Campus Lübeck, 23538 Lübeck (DE)
(72) Inventor: SCZAKIEL, Georg, 23627 Gross-Grönau (DE); HOPERT, Anne, 23564 Lübeck (DE); WÜNSCHE, Winfried, 23562 Lübeck (DE); OVERHOFF, Marita, 23552 Lübeck (DE)
(74) Representative: Müller-Boré & Partner Patentanwälte
(86) International application number: PCT/EP2004/010162
(87) International publication number: WO 2005/024033

(56) References cited:
- WO-A-01/08707
- US-A- 5 585 263
- LANGLE-ROUAULT F ET AL: "UP TO 100-FOLD INCREASE OF APPARENT GENE EXPRESSION IN THE PRESENCE OF EPSTEIN-BARR VIRUS ORIP SEQUENCES AND EBNA1: IMPLICATIONS OF THE NUCLEAR IMPORT OF PLASMIDS" JOURNAL OF VIROLOGY, vol. 72, no. 7, July 1998 (1998-07), pages 6181-6185, XP002924894 ISSN: 0022-538X -& DATABASE GENBANK [Online] 6 December 1983 (1983-12-06), "Epstein-Barr virus (EBV) genome, strain B95-8." XP002317033 retrieved from NCBI Database accession no. V01555
- MARCELLO MARCO ET AL: "Selecting karyophilic DNA cis elements in Xenopus laevis oocytes; a new approach" INTERNATIONAL JOURNAL OF DEVELOPMENTAL BIOLOGY, vol. 46, no. 3, May 2002 (2002-05), pages 309-316, XP008042714 ISSN: 0214-6282
- FAR ROSEL KRETSCHMER-KAZEMI ET AL: "The activity of siRNA in mammalian cells is related to structural target accessibility: A comparison with antisense oligonucleotides." NUCLEIC ACIDS RESEARCH, vol. 31, no. 15, 1 August 2003 (2003-08-01), pages 4417-4424, XP002317031 ISSN: 0305-1048
- WACHECK VOLKER ET AL: "Antitumor effect of G3139 Bcl-2 antisense oligonucleotide is independent of its immune stimulation by CpG motifs in SCID mice." ANTISENSE AND NUCLEIC ACID DRUG DEVELOPMENT, vol. 12, no. 6, December 2002 (2002-12), pages 359-367, XP002317032 ISSN: 1087-2906

## Description

The present invention relates to a novel system for the trans-membrane transport including the cellular uptake/delivery of nucleic acids, substances that contain a nucleic acid component, recombinant genetic material or substances that can be attached to a nucleic acid, in biological systems. In particular, the present invention relates to the use of a nucleic acid comprising a first nucleotide sequence having a specific signal sequence, wherein said signal sequence increases or causes the uptake of the nucleic acid into a cell.

A large number of tools have been developed during the recent years for introducing recombinant genetic material (for example recombinant DNA) or oligomeric nucleic acid drugs (for example antisense oligonucleotides, CpG-immunostimulatory oligonucleotides, siRNA/RNAi, ribozymes, and aptamers) into cells. The different techniques can be divided into two categories. The first category relates to physical techniques such as microinjection, electroporation, particle bombardment etc.. The second category involves techniques relating to molecular and cell biology, wherein genetic material to be transferred is combined with a biological or synthetic vector which promotes the introduction of said genetic material into a cell.

The vectors which are currently most effective, are viral vectors, such as adenoviral or retroviral vectors. The techniques which have been developed are based on the natural properties which these viruses possess for e.g. traversing cell membranes, evading degradation of their genetic material, enabling their genome to penetrate into the nucleus and integrating into the genetic material of the host cells. Nevertheless, the viral approach suffers from a large number of drawbacks such as the risk of infectious viral particles, the risk of artefactual mutagenesis resulting from insertion in the host cell and difficulties in biological control.

Additionally, non-viral methods are available, for example coprecipitation with calcium phosphate, use of cationc lipids or Lipofectamine etc.. However, these techniques suffer from a number of limitations, in particular their low level of *in vivo* efficacy.

Moreover, the viral and non-viral techniques known in the art are time consuming and cost extensive.

Further, Längle-Rouault et al. (Journal of Virology, July 1998, pp. 6181-6185) describe *inter alia* an oriP-specific transport of plasmid DNA from the cytoplasm of cells to the nucleus, and US-patent 5,558,263 describes a novel retroviral nucleotide sequence comprising a constitutive transport enhancer which functions to transport mRNA transcripts from the nucleus to the cytoplasm of a cell.

Therefore, systems that cause the uptake of a nucleic acid, e.g. a DNA, into a cell via a nucleic acid transport mechanism are highly desired. However, up to now such systems (e.g. DNA-*cis*-elements) are not known.

Thus, the technical problem underlying the present invention is to provide a novel system for transferring nucleic acids, e.g. specific DNA constructs, into a cell of interest.

The solution to the above technical problem is achieved by the embodiments characterized in the claims.

In particular, the present invention relates to the use of a nucleic acid comprising a first nucleotide sequence having the signal sequence unit 5'-(TCGTGT)ₙ -3' (6mer) wherein n is an integer ranging from 1 to 10, more preferably from 1 to 5 and most preferably from 1 to 3 said signal sequence causing a trans-membrane transport such as the uptake/delivery of the nucleic acid in biological systems such as cells and subcellular compartments. If n is greater than 1 (i.e. at least two identical units of the signal sequences) one or more spacing nucleotides may be present between the units, wherein said spacing nucleotides do not negatively affect the biological function of the signal sequence. The first nucleic acid sequence has preferably a lenght ranging from 3 to 150, more preferably 3 to 120, and most preferably from 3 to ≤ 20 nucleotides. The signal sequence may be located on the 5'-and/or 3'-ends of the nucleic acid. Further, the signal sequence has preferably a distance from the 5'-and/or 3'-ends of the nucleic acid ranging from 0 to 30, more preferably 0 to 20 and most preferably 0 to 15 nucleotides, wherein said nucleotides do not negatively affect the biological function of the signal sequence. The first nucleotide sequence may also exhibit at least one internucleotide-phosphorothioate linkage within and/or outside the signal sequence.

The nucleic acid of the present invention further contains at least one second nucleotide sequence to be transported and/or one or more components covalently linked and/or forming a complex with the first and/or second nucleotide sequence.

The term "second nucleotide sequence to be transported" encompasses a heterologous or homologous recombinant nucleotide sequence comprising, e.g. a coding region for at least a polypeptide and/or a RNA transcript of interest such as an antisense nucleic acid, and/or a ribozyme, and/or one or more control regions and/or partially or completely double stranded RNA such as siRNA and/or one or more aptamers and one or more nucleic agent e.g. immunomodulating agents such as CpG-oligonuceotides. This term includes also the use of the second nucleotide to be transported as an oligomeric nucleic acid drug" such as chemically synthesized nucleic acids as for example antisense oligonucleotides, siRNA, CpG oligonucleotides, ribozymes, aptamers, intramers, triple helix-forming oligonucleotides, etc.)

The term "a coding region for at least a polypeptide" means that the corresponding nucleotide sequence comprises at least one open reading frame (OFR) or one or more exons, respectively, wherein if necessary untranslated regions such as introns are inserted, and the ORF or the exons is (are) coding for e.g. the primary structure of a polypeptide. The coding region for at least a polypeptide can be homologous or heterologous relating to the cell. Additionally, the term "a coding region for at least an antisense nucleic acid" means that the corresponding nucleotide codes for a nucleic acid that is capable of binding to a target RNA thereby inhibiting the biological function of the RNA, e.g. the translation of an mRNA. The term "a coding region for at least one ribozyme" means that the corresponding nucleotide sequence codes for a nucleic acid that can cleave a target mRNA thereby inhibiting its biological function. Preferably such a ribozyme is a hammerhead ribozyme. The term "a coding region for at least one siRNA" means the corresponding nucleotide sequence codes for a nucleic acid that can *inter alia* mediate catalytically the degradation of mRNA.

The term "control region" comprises all nucleotide sequences that participate in gene regulation, i.e. the regulation of the expression of a gene, such as promoters, enhancers, operators, transcription/translation signals and polyadenylation signals.

The term "promoter" indicates that part of the nucleotide sequence that fixes the initiation point and the initiation frequency (mRNA synthesis) of the transcription of a gene. The term "enhancer" indicates that part of the nucleotide sequence that increases the efficiency of the transcription. Enhancers occur in e.g. eukaryotic viruses and cellular genes. The cis-active enhancers can show an effect over a distance of several kilobases in the nucleotide sequence. They function in both orientations and not dependent on their position, i.e. they can be found in the 5'-region or in the 3'-region of a gene to be controlled, moreover they can be localized in an intron. The term "operator" indicates that part of the nucleotide sequence wherein the regulatory proteins (for example repressors and activators) can bind, thereby blocking (negative regulation) or activating (positive regulation) adjacent structural genes, such as the coding region for a polypeptide. The term "transcription/translation signal" comprises particularly control regions in the 5'-flanking region of the nucleotide sequence coding for a polypeptide, such as TATA- and CCAT-boxes, so called "Kozack" sequences (M. Kozack (1991) "An analysis of vertebrate mRNA sequences: intimations of translational control", J.Cell.Biol. 115(4), 887-903) as well as ribosomal binding sites and IRES (internal ribosomal entry sites)-elements. The term "polyadenylation site" indicates nucleotide sequences that are responsible for adding a poly(A)tail to a mRNA and for processing the 3'-end of the mRNA. The polyadenylation signal for the addition of a poly(A) tail in a mammal contains for example two elements: an AAUAAA-sequence 20 to 30 nucleotides in front and a diffuse GU-rich sequence after the 3'-end of that part of the nucleotide sequence that codes for the mRNA.

The term "biologically active component" comprises all biological structures that are based on single or several molecules of all substance classes, except nucleic acids, for example peptides such as polypeptides or proteins, lipids such as phospholipids and saccharide such as sugars or oligo- and polysaccharides. All of the biologically active components can further contain co-factors, prosthetic groups and ions etc..

According to a preferred embodiment of the above-defined nucleic acid, the control region(s) is (are) capable of regulating the expression of the coding region of at least one polypeptide, antisense nucleic acid, ribozyme and siRNA. The term "regulating the expression of the coding region" indicates that the control region(s) is (are) capable to control the transcription and/or the translation of at least one polypeptide, antisense nucleic acid, ribozyme and siRNA in a positive or negative manner.

The terms "biological system", "cell" and "cell of interest" according to the present invention encompass prokaryotic cells, yeast cells and eukaryotic cells, plant cells and mammalian cells. In addition, cells can be used for the *in vivo* treatment (e.g. gene therapy) of a mammal including humans, wherein for example the cells are isolated from the patient suffering from a disease, treated with nucleic acid and then returned to the patient. Further, this term includes transgenic organism such as a transgenic non-human mammal (not including human).

The term "vector" according to the present invention encompasses a DNA- and/or RNA-replicon that can be used for the amplification and/or expression of the nucleotide sequence of the above-defined nucleic acid. The vector may contain one or more of the above-defined control regions and/or nucleotide sequences in the 5'- and/or 3'-region of the nucleotide in addition further nucleotide sequences in the 5' and/or 3'-region of the above defined nucleotide sequence coding for amino acid sequences that are useful for detecting and isolating proteins such as a polypeptide that is encoded by the above-defined nucleotide sequence. Preferably, the vector contains further elements that are responsible for the integration stability of the above-defined nucleic acid coding for at least one polypeptide, antisense nucleic acid, ribozyme or siRNA into the genome of the host organism and/or the transient expression of the above-defined nucleotide sequence. Additionally, the vector may preferably contain selectable genes such as one or more antibiotic resistant genes that enable an easy selection of the cells that have integrated the vector

The term "host organism" comprises cells of fungus, plants, mammals (i.e. animals and humans) as well as parts of these cells.

A method for producing the above-defined nucleic acid or the above-defined vector comprises the steps of:
(a) cultivating the host organism in a suitable medium under suitable conditions, and
(b) isolating the desired product from the medium and/or the host organism.

The nucleic acid defined above can be used to provide a pharmaceutical composition containing the above defined nucleic acid and/or the above-defined vector and/or the above-defined host organism and optionally a pharmaceutical acceptable carrier and/or diluent. In the pharmaceutical composition the signal sequence that causes the trans-membrane transport such as the cellular uptake of the nucleic acid can be combined with at least one antisense nucleic acid, siRNA, ribozyme or sequence regions of nucleic acid agents such as e.g. immunomodulating agents such as CpG-oligonucleotides.

The present invention discloses a novel system for introducing particularly nucleic acids of interest, for example recombinant DNA or long-chain and/or oligomeric single- and double-stranded nucleic acids, into a cell, for example a mammalian cell by using novel nucleic acid cis-elements that can replace the transport function of commonly used viral or non-viral vectors. Such nucleic acid cis-elements can cause the uptake of for example naked, double stranded or partially or completely single stranded DNA into a mammalian cell thereby representing the easiest transfer of nucleic acids into cells of interest. By this novel system the time consuming complexation of recombinant DNA via viral or non-viral vector components in biomedicine can be substantially supported or replaced. It is also possible for new approaches in molecular medicine to combine a recombinant gene of interest such as those used for gene therapy, with the above-defined DNA transport elements and use such a construct directly. This method results in a drastic simplification of the protocols used in somatic gene therapy and the *in vivo* application of oligomeric nucleic acid drugs.

The figures show:
- Fig. 1: is in a bar graph showing the relative uptake of nucleic acids in BHK cells after treatment with oligonucleotides containing a nucleotide sequence having the signal sequence 5'-TCGTGT-3' or modifications thereof.
- Fig. 2: is a bar graph showing the relative uptake of nucleic acids in BHK-21 cells after treatment with oligonucleotides containing a nucleotide sequence having a single base mutation in the signal sequence 5'-TCGTGT-3'.
- Fig. 3: is a bar graph showing the immunostimulation (relative CD 54 expression) in BJAB cells after treatment with CpG-oligonucleotides containing a nucleotide sequence having the signal sequence 5'-TCGTGT-3'.
- Fig. 4: is a bar graph showing the relative ICAM-1 (CD 54) expression in human ECV304 cells after treatment with a siRNA sequence directed against ICAM-1 linked to the nucleotide sequence having the signal sequence 5'- TCGTGT-3'.
- Fig.5: is a graph showing the relative ³²P-signal (corresponding to the relative uptake) of siRNA constructs si2B-3E' and si2B-CH12 in human ECV304 cells depending on the concentration (x-axis); - ◆ - represents the si2B- 3E'construct; -■- represents the si2B-CH12 construct.

The following examples illustrate the present invention in more detail. However, the present invention is not limited by these examples. In the examples and figures "s" means an internucleotide-phosphorothioate linkage.

### Examples

### Example 1: Uptake of oligonucleotides containing the signal sequence of the present invention

Nucleic acids, one containing the selected signal sequence 5'-TCGTGT-3' (Table 1: "157") or derivatives thereof and controls are coupled to ³²P at the 5'-terminus. An amount of 10 pmole of oligonucleotide was 5'-labelled by [γ-³²P]ATP and polynucleotide kinase. A number of 5 x 10⁵ of BHK cells was seeded semi-confluently (6 cm dishes), grown overnight and treated subsequently with 1.8 nM ³²P-labelled oligonucleotides for 2 hours. Then cells were washed twice with PBS, removed from the culture dishes by trypsin, washed again two times with PBS containing 1% of BSA, then washed once with a buffer containing 0.5M NaCl and 0.2 M acetate buffer pH 2.5 and re-washed twice with PBS. Cells were pelleted by centrifugation and lysed by sonication. The cell debris was cleared by centrifugation and the supernatant was extracted by phenol/chloroform. DNA including oligonucleotides was precipitated by ethanol and analysed by polyacrylamidegel electrophoresis under denaturing conditions. Band intensities were measured and quantified by a phosphorimaging system. Table 1 shows the nucleic acids used for the quantitative comparison of the uptake.

**Table 1:**

| **Name** | **Sequences** | **Comment** |
|---|---|---|
| CH1 | 5'-GsAsTsAGCACAG TCTAGATCAGCGGCCGCAATAsCsAsA-3' | complementary |
| CH2 | 5'-GsAsTsCGTGCCG TCTAGATCAGCGGCCGCAATAsCsAsA-3' | |
| CH3 | 5'-GsAsTsCCTGCGG TCTAGATCAGCGGCCGCAATAsCsAsA-3' | scrambled |
| CH4 | 5'-CsCsGsTGTCGTG TCTAGATCAGCGGCCGCAATAsCsAsA-3' | 40 + 2x CGTG |
| CH5 | 5'-GsAsTsAGCTGAG TCTAGATCAGCGGCCGCAATAsCsAsA-3' | T → A |
| CH6 | 5'-GsAsTsTAAAATG TCTAGATCAGCGGCCGCAATAsCsAsA-3' | CGTG → AAAA |
| CH7 | 5'-GsAsTsTCTGCTG TCTAGATCAGCGGCCGCAATAsCsAsA-3' | scrambled |
| CH8 | 5'-AsAsAsTCGTGTG TCTAGATCAGCGGCCGCAATAsCsAsA-3' | cis-element |
| CH9 | 5'-GsCsCsTGTGCCG TCTAGATCAGCGGCCGCAATAsCsAsA-3' | Pool ODN |
| 157 | 5'-GsAsTsTCGTGTG TCTAGATCAGCGGCCGCAATAsCsAsA-3' | positive control |
| 40 | 5'-AsAsAsAGAGTTG TCTAGATCAGCGGCCGCAATAsCsAsA-3' | negative control |

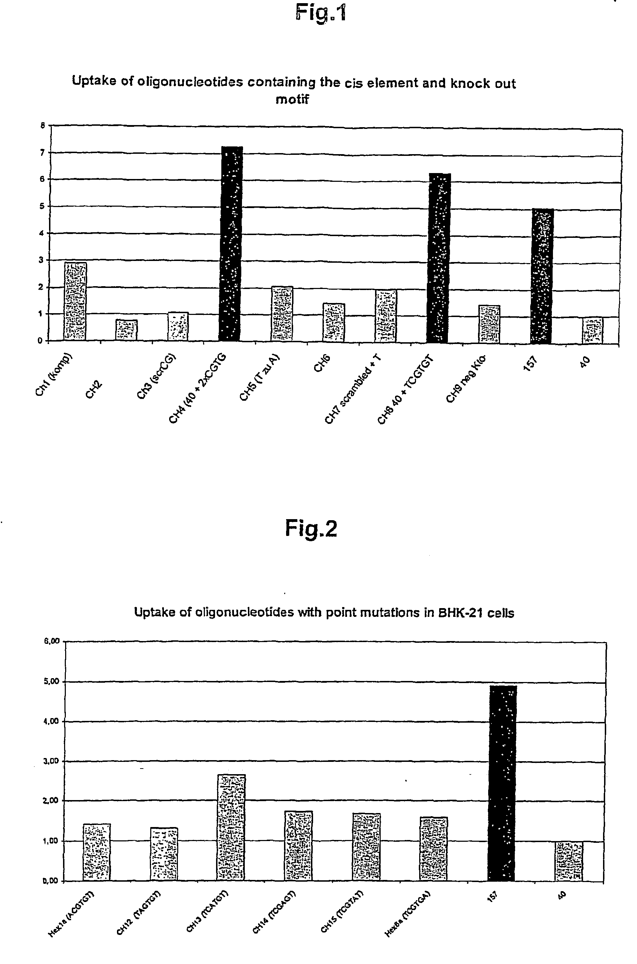

Figure 1 clearly demonstrates that the oligonucleotides 157, CH4 and CH8 containing the signal sequence according to the invention show a significant higher uptake into the cell than the control oligonucleotides CH1, CH2 CH3, CH5, CH6, CH7, CH9 and 40.

### Example 2: Uptake of oligonucleotides with point mutations in the signal sequence

Example 2 is carried out as Example 1. The nucleic acids contain single base mutations in the signal sequence 5'-TCGTGT-3'. Table 2 shows the nucleic acids used for the quantitative comparison of the uptake.

**Table 2:**

| **Name** | **Sequences** | **Comment** |
|---|---|---|
| Hex 1a | 5'-GsAsTsACGTGTG TCTAGATCAGCGGCCGCAATAsCsAsA-3' | complementary |
| CH12 | 5'-GsAsTsTAGTGTG TCTAGATCAGCGGCCGCAATAsCsAsA-3' | |
| CH13 | 5'-GsAsTsTCATGTG TCTAGATCAGCGGCCGCAATAsCsAsA-3' | scrambled |
| CH14 | 5'-GsAsTsTCGAGTG TCTAGATCAGCGGCCGCAATAsCsAsA-3' | 40 + 2x CGTG |
| CH55 | 5'-GsAsTsTCGTATG TCTAGATCAGCGGCCGCAATAsCsAsA-3' | T → A |
| Hex 6a | 5'-GsAsTsTCGTGAG TCTAGATCAGCGGCCGCAATAsCsAsA-3' | CGTG → AAAA |
| 157 | 5'-GsAsTsTCGTGTG TCTAGATCAGCGGCCGCAATAsCsAsA-3' | positive control |
| 40 | 5'-AsAsAsAGAGTTG TCTAGATCAGCGGCCGCAATAsCsAsA-3' | negative control |

Figure 2 clearly demonstrates that the oligonucleotide 157 containing the signal sequence according to the invention shows a significant higher uptake into the cell than the oligonucleotides Hex 1a, CH12, CH13, CH14, CH15, Hex 6a and 40.

### Example 3: Immunostimulation in BJAB cells using oligonucleotides containing the signal sequence

The signal sequence 5'-TCGTGT-3' is coupled to the 3'-terminus to CpG immunostimulating active oligonucleotide G3139 that increases the expression of ICAM-1 (CD 54) Then BJAB cells (B-lymphoid cells) are incubated with these nucleic acid mixture for 40 h with oligonucleotides containing the CpG motive with or without an additional uptake sequence motive or with a mutated form of the sequence motive at a final concentration of 250 nM. BJAB cells were harvested, washed with phosphate-buffered saline (PBS; pH 7,4) and incubated with a phycoerythrin (PE)-conjugated CD54 monoclonal antibody (clone LB-2) or an isotype-identical PE control (both Becton Dickinson, Heidelberg) in 50 µl PBS 1% BSA for 1h at 4°C. After antibody incubation cells were washed again with PBS and resuspended in 500 µl 1% paraformaldehyde in PBS pH 7,4. ICAM-1 expression was analysed using a FACSCalibur and CellQuest Pro software (Becton Dickinson, Heidelberg). Table 3 shows the nucleic acids used for the immunostimulation in BJAB cells.

**Table 3:**

| **Name** | **Sequences** | **Comment** |
|---|---|---|
| G3139 | 5'-TsCsTsCsCsCsAsGsCsGsTsGsCsGsCsCsAsT-3' | |
| G3139 157 | 5'-TsCsTsCsCsCsAsGsCsGsTsGsCsGsCsCsAsTGATTCGTsGsTsG-3' | pos. control |
| G3139 40 | 5'-TsCsTsCsCsCsAsGsCsGsTsGsCsGsCsCsAsTAAAAGAGsTsTsC-3' | neg. control |
| G3139CH1 | 5'-TsCsTsCsCsCsAsGsCsGsTsGsCsGsCsCsAsTGATAGCAsCsAsG-3' | neg. control |

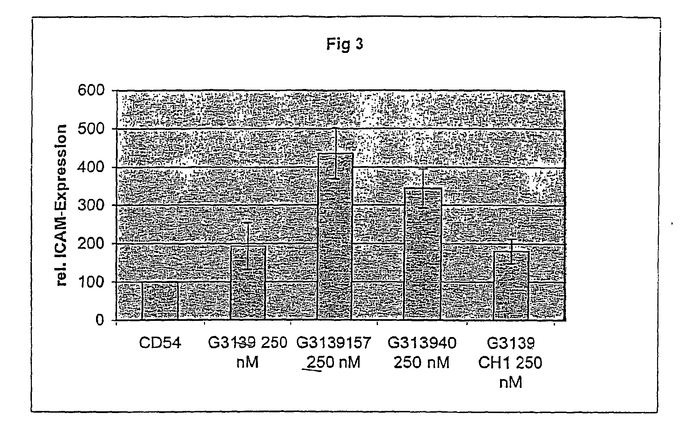

Figure 3 clearly demonstrate that the oligonucleotide G3139 157 containing the signal sequence according to the invention leads to a higher immunostimulation of the BJAB cells than the oligonucleotides G3139, G3139 40 and G3139CH1. The first bar "CD54" in figure 3 shows the expression level of CD54 in the absence of any oligonucleotide, i.e. the basal expression level.

### Example 4: ICAM-1 expression after treatment with siRNA linked to the signal sequence

The signal sequence 5'-TCGTGT-3' is coupled to a siRNA sequence that is directed against ICAM-1 (CD54). A scrambled siRNA is used as a control (si-sc). ECV304 cells were seeded at a density of 1.25 x 10⁵ cells/ well of a 12well plate. After 15 h cells were washed with Opti-MEM I medium (Invitrogen, Netherlands). Then siRNAs were added at a concentration of 1 µM in Opti-MEM I medium for 2h. ECV304 cells were washed with PBS, trypsinated with 0.25% trypsin 0.02% EDTA in PBS after stimulation with 200 U, 100 U and 50 U of IL-1β for 4h respectively. Then ECV304 were harvested and prepared for FACS analysis as described above. Table 4 shows the siRNAs used in this assay.

**Table 4:**

| **Name** | **Sequences** | **Comment** |
|---|---|---|
| si2B-E3' | 5'-GCCUCAGCACGUACCUCUAgattcgtgstsgs | sense |
| | 3'-ttCGGAGUCGUGCAUGGAGAU | antisense |
| si-sc | 5'-CGAACUCACUGGUCUGACCdtdt-3' | sense |
| | 3'-dtdtGCUUGAGUGAACCAGACUGG-5' | antisense |

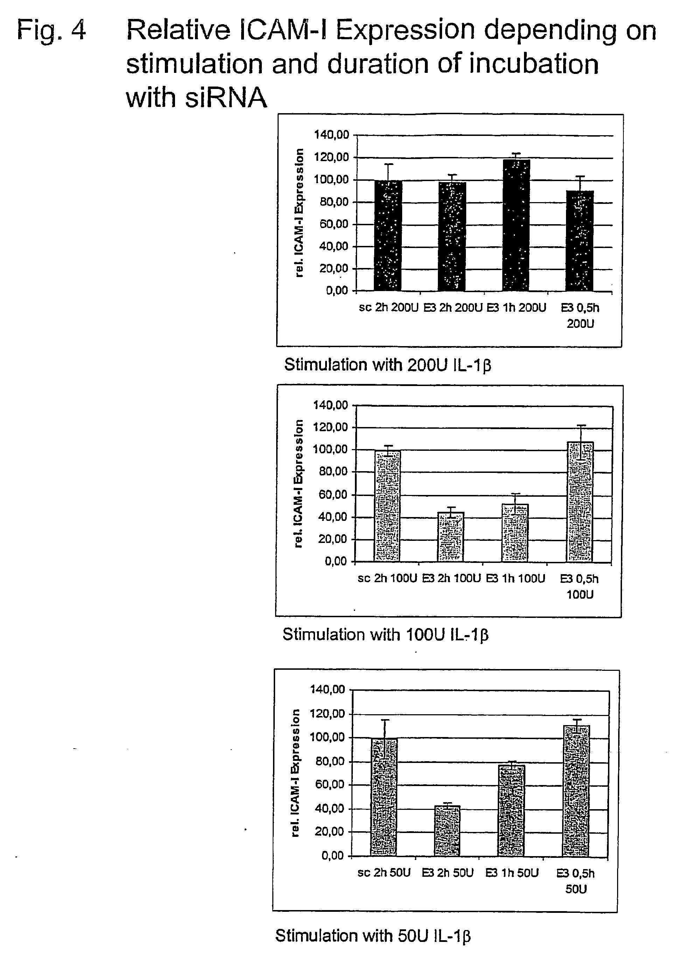

Figure 4 clearly demonstrates that the ICAM-1 expression is significantly reduced in cells containing the linked siRNA, depending on the stimulation and duration of the incubation.

### Example 5: Selection process of specific signal sequence of the invention

For the selection procedure 1 x 10⁷ BHK-21 cells were seeded in cell culture flasks (175 cm²), after overnight incubation cells were washed with PBS and treated with 3 µg pool oligonucleotides in DMEM/F12 without FCS for 2 h. Thereafter BHK-21 cells were washed with PBS twice, trypsinated, washed with PBS 1%BSA twice, once with 0,5 M NaCl, O,2 M acetic acid, pH 2,5 and then washed twice with PBS. The pellet was resuspended in 100 µl TE and the cells were lysed by ultrasonication. The cell debris was pelleted by two centrifugation steps and the supernatant was used for phenol/chloroform extraction and ethanol precipitation of DNA. The DNA in the lysate was kinased as follows: 2 µl lysate, 10 U T4 polynucleotide kinase, 2 µl 10x reaction buffer A (forward reaction), 2 µl 10 mM ATP and 13 µl H₂O were incubated 30 min at 37°C and for 10 min at 65°C. This reaction volume was purified using nick columns according to manufacturer instructions. The eluate was vacuum dried and resuspended in 6 µl of H₂O. Different primers were ligated to 6 µl of the kinased DNA in the following reaction: 1 µl primer 0.5 pmol/µl, 1µl T4 RNA ligase, 2µl T4 RNA ligase buffer and 10 µl 50% PEG 8000. 2 µl of the ligation were used in a PCR amplification containing 1x PCR buffer, 1 U Taq polymerase, 0-.5 µM Primer PB, 0.5 µM Primer X (used in ligation reaction), 50 nM each dNTP ad 50 µl H₂O with the following cycling parameters 94°C 3 min 1x, 94°C 30 s, 55°C 50 s, 72°C 1 min each 27x, 72°C 5 min 1x, 4°C 24 h. PCR products were analysed by 2 % ethidium bromide stained agarose gels. 2 µl of the PCR product was cloned into a TA cloning vector according to manufacturer instructions and were grown in E. coli DH5. 50 clones were sequenced. Additionally the phosphorylation, ligation, PCR and cloning into the TA has been done with pool 0 oligonucleotides for comparison of diversity. 41 selected sequences have been obtained and were synthesized by Interactiva, pooled again and used for a second round of selection as described above. From this round of selection 40 clones were sequenced and these sequences were compared and analysed for 3mer, 4mer and 5mer motifs with the sequences obtained by cloning of pool 0 by a computer program kindley provided by Olga Matveeva (Salt Lake City, U.S.A.).

The following Table 5 lists several nucleotide sequence motifs that have been selected for their ability to signal cellular uptake by mammalian cells (BHK cells) in a statistically significant manner according to the protocol described above.

**Table 5:**

| | **Sequence** | **present in active** | **present in non-active** | **p-value** |
|---|---|---|---|---|
| 3mer | CGT | 16 | 3 | 0.00004 |
| | TCG | 15 | 5 | 0.00101 |
| 4mer | TCGT | 12 | 1 | 0.0001 |
| | CGTG | 15 | 1 | 0.00001 |
| 5mer | TCGTG | 11 | 0 | 0.00004 |
| 6mer | TCGTGT | 4(6) | 0 | |

### Example 6: Cellular uptake of siRNA linked to the signal sequence or to the mutated signal sequence, respectively

A siRNA sequence that is directed against ICAM-1 (CD54) is coupled to the signal sequence 5'-TCGTGT-3' (si2B-E3') or to the point mutated signal sequence 5'-TAGTGT-3' (si2B-CH12).

EVC304 cells were seeded at a density of 3 x 10⁵ cells/well of a 6 well plate. After 16 h cells were washed twice with pre-warmed PBS (room temperature) and incubated with different amounts of si2B-E3' or si2B-CH12 in a volume of 1 ml OptiMEM (Gibco-Invitrogen, Karlsruhe, Germany). After incubation for 2h the cells were washed five times with pre-warmed PBS and trypsin-treated with 0.25% trypsin 0.02% EDTA in PBS. The cells were lysed with TE buffer pH 7.4 containing 1% NP-40 for 10 min at 0°C followed by RNA-phenol-chloroform extraction and precipitation with ethanol. The RNA pellets were resuspended in a hybridisation buffer followed by a RNase-protection assay. For the RNase-protection assay 10µl of the RNA were hybridized to 40 fmole of ³²P-sense-2B followed by treatment with RNaseT1/RNaseA. Samples were analyzed by polyacrylamid gel electrophoresis. The quantification of the signals was performed by a Phophsolmager and the software "ImageQuant". Table 6 shows the siRNAs used in this assay.

**Table 6:**

| **Name** | **Sequences** | **Comment** |
|---|---|---|
| si2B-E3' | 5'-GCCUCAGCACGUACCUCUAgat**tcgtgsts**gs | sense |
| | 3'-ttCGGAGUCGUGCAUGGAGAU | antisense |
| si2B-CH12 | 5'-GCCUCAGCACGUACCUCUAgat**tagtgsts**gs | sense |
| | 3'-ttCGGAGUCGUGCAUGGAGAU | antisense |

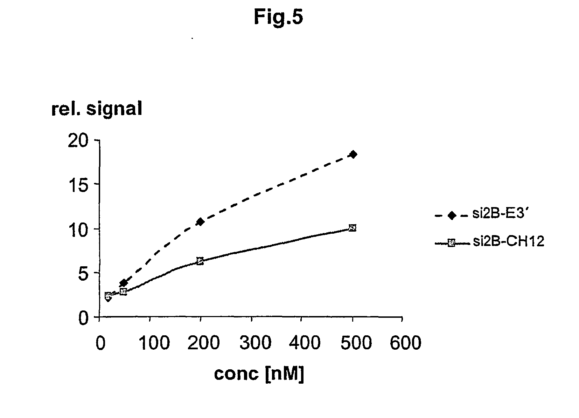

Figure 5 clearly demonstrates that the cellular uptake of the siRNA containing the signal sequence according to the invention compared with the cellular uptake of the siRNA containing the point mutated signal sequence, is significantly increased, depending on the concentration of the respective siRNA during the incubation.

### Example 7: ICAM-1 expression of either transfected or non-tranfected cells after treatment with different siRNA constructs

A siRNA sequence that is directed against ICAM-1 (CD54) (si2B) is coupled to the signal sequence 5'-TCGTGT-3' (si2B-E3') or to the point mutated signal sequence 5'-TAGTGT-3' (si2B-CH12). A scrambled siRNA is used as a negative control (si-scr). Table 7 shows the siRNA constructs used in this assay.

**Table 7:**

| **Name** | **Sequences** | **Comment** |
|---|---|---|
| si2B | 5'-GCCUCAGCACGUACCUCUAdtdt-3' | sense |
| | 3'-ttCGGAGUCGUGCAUGGAGAU-5' | antisense |
| si2B-E3' | 5'-GCCUCAGCACGUACCUCUAgat**tcgtgsts**gs | sense |
| | 3'-ttCGGAGUCGUGCAUGGAGAU | antisense |
| si2B-CH12 | 5'-GCCUCAGCACGUACCUCUAgat**tagtgsts**gs | sense |
| | 3'-ttCGGAGUCGUGCAUGGAGAU | antisense |
| si-scr | 5'-CGAACUCACUGGUCUGACCdtdt-3' | sense |
| | 3'-dtdtGCUUGAGUGAACCAGACUGG-5' | antisense |

ECV304 cells were seeded at a density of 1.5 x 10⁵ cells/well of a 12 well plate 16 h prior to the transfection experiment or to the uptake experiment, respectively. Cells were washed twice with PBS. For the transfection experiment cells were incubated for 4h with 10 nM of the siRNA constructs with the transfection reagent Lipofectamin 2000 (Invitrogen, Germany) following the costumer's instructions. Then the cells were washed with PBS, followed by incubation with 50U of IL-1β for 14h in medium 199 (Gibco-Invitrogen, Karlsruhe, Germany) supplemented with 10% FCS. For the uptake experiment cells were incubated with 200 nM of the siRNA constructs for 2h in a final volume of 400 µl followed by stimulation with 50U IL-1β for 4h. Then the cellular RNA was prepared for the RT-PCR with Rneasy-MiniKit (Qiagen, Hilden, Germany) and transcribed with the TaqMan®Reverse Transcription Reagents (Applied Biosystems, Germany). The quantitative PCR was accomplished according to manufacturer's instructions with SYBR green PCR core reagent (Eurogentec, Seraing, Belgium). For the detection of ICAM-1 cDNA the following primers were used: forward primer 5'-GCCACTTCTTCTGTAAGTCTGTGGG-3' and the reverse primer 5'-CTACCGGCCCTGGGACG-3'. To standardize the samples, a Q-PCR for GAPDH was performed (forward primer 5'-AACAGCGACACCCACTCCTC-3'; reverse primer 5'-GGAGGGGAGATTCAGTGTGGT-3'). The ICAM-1 expression of the si-scr treated cells was set at 100%. Table 8 show the results of these experiments.

**Table 8:**

| | **relative ICAM-1 expression (%)** | |
|---|---|---|
| **construct** | **spontaneous uptake** | **transfection** |
| si2B | 100 | 7 |
| si-scr | 100 | 100 |
| si2B-E3' | 75 | 20 |
| si2B-CH12 | 117 | 16 |

Table 8 clearly demonstrates that the constructs si2B, si2B-E3'and si2B-CH12 inhibit the ICAM-1 expression after transient transfection of the cells, whereas the si-scr construct exhibits no inhibitory effect. However, in the uptake experiment only the construct si2B-E3' containing the signal sequence according to the invention shows an inhibition of ICAM-1 expression.

## Claims

1. Use of a nucleic acid comprising a first nucleotide sequence having the signal sequence unit 5'-(TCGTGT)ₙ-3', wherein n is an integer ranging from 1 to 10, for a trans-membrane transport into a cell, caused by said signal sequence, wherein said trans-membrane transport is not practiced on the human or animal body, and wherein the nucleic acid contains further at least one second nucleotide sequence to be transported.

2. The use according to claim 1, wherein the nucleic acid contains further one or more components covalently linked and/or forming a complex with the first and/or second nucleotide sequence, wherein the covalently linked and/or complexed components are biologically active and no nucleic acids.

3. The use according to claims 1 or 2, wherein the second nucleotide sequence comprises at least one or more control regions and/or a coding region, wherein the coding region codes for at least one polypeptide, antisense nucleic acid, ribozyme or siRNA.

4. The use according to claim 3, wherein the control region comprises a promoter and/or an enhancer and/or an operator and/or a transcription/translation signal and/or a polyadenylation signal.

5. The use according to claims 3 or 4, wherein the control region(s) is (are) capable of regulating the expression of the coding region for at least one polypeptide, antisense nucleic acid, ribozyme or siRNA.

6. The use according to any preceding claim, wherein the cell is a mammalian cell.

## Patentansprüche

1. Verwendung einer Nukleinsäure, umfassend eine erste Nukleotidsequenz, welche die Signalsequenzeinheit 5'-(TCGTGT)ₙ-3' aufweist, wobei n eine ganze Zahl, die von 1 bis 10 reicht, ist, für einen Transmembrantransport in eine Zelle, der von der Signalsequenz verursacht wird, wobei der Transmembrantransport nicht am menschlichen oder tierischen Körper vorgenommen wird, und wobei die Nukleinsäure weiter mindestens eine zweite, zu transportierende Nukleotidsequenz enthält.

2. Verwendung nach Anspruch 1, wobei die Nukleinsäure weiter einen oder mehrere Bestandteil(e) enthält, der/die kovalent mit der ersten und/oder zweiten Nukleotidsequenz verbunden ist/sind, oder einen Komplex damit bilde(t/n), wobei die kovalent verbundenen und/oder komplexierten Bestandteile biologisch aktiv sind und keine Nukleinsäuren sind.

3. Verwendung nach Anspruch 1 oder 2, wobei die zweite Nukleotidsequenz mindestens einen oder mehrere Kontrollbereich(e) und/oder einen kodierenden Bereich umfaßt, wobei der kodierende Bereich mindestens ein Polypeptid, eine antisense-Nukleinsäure, ein Ribozym oder eine siRNA kodiert.

4. Verwendung nach Anspruch 3, wobei der Kontrollbereich einen Promotor und/oder einen Enhancer und/oder einen Operator und/oder ein Transkriptions-/Translationssignal und/oder ein Polyadenylierungssignal umfaßt.

5. Verwendung nach Anspruch 3 oder 4, wobei die Kontrollregion(en) befähigt ist/sind, die Expression des kodierenden Bereichs für mindestens ein Polypeptide, eine antisense-Nukleinsäure, ein Ribozym oder eine siRNA zu regulieren.

6. Verwendung nach einem der vorhergehenden Ansprüche, wobei die Zelle eine Säugerzelle ist.

## Revendications

1. Utilisation d'un acide nucléique comprenant une première séquence de nucléotides ayant l'unité de séquence de signal 5'-(TCGTGT)ₙ-3', dans laquelle n est un entier dans une gamme de 1 à 10, pour un transport transmembranaire dans une cellule, causé par ladite séquence de signal, dans laquelle ledit transport transmembranaire n'est pas pratiqué sur le corps humain ou celui d'un animal, et dans laquelle l'acide nucléique contient par ailleurs au moins une deuxième séquence de nucléotides à transporter.

2. L'utilisation selon la revendication 1, dans laquelle l'acide nucléique contient par ailleurs un ou plusieurs composants liés de façon covalente et/ou formant un complexe avec la première et/ou la deuxième séquence de nucléotides, dans laquelle les composants liés de façon covalente et/ou complexés sont biologiquement actifs et non pas des acides nucléiques.

3. L'utilisation selon la revendication 1 ou 2, dans laquelle la deuxième séquence de nucléotides comprend au moins une ou plusieurs régions de contrôle et/ou une région de codage, dans laquelle la région de codage code pour au moins un polypeptide, acide nucléique antisens, ribozyme ou ARN interférent.

4. L'utilisation selon la revendication 3, dans laquelle la région de contrôle comprend un signal promoteur et/ou amplificateur et/ou opérateur et/ou de transcription/traduction et/ou un signal de polyadénylation.

5. L'utilisation selon la revendication 3 ou 4, dans laquelle la(les) région(s) de contrôle est(sont) capable(s) de réguler l'expression de la région de codage pour au moins un polypeptide, acide nucléique antisens, ribozyme ou ARN interférent.

6. L'utilisation selon l'une quelconque des revendications précédentes, dans laquelle la cellule est une cellule de mammifère.
